(19) Europäisches Patentamt
European Patent Office
Office européen des brevets

(11) Veröffentlichungsnummer: **0 196 025 A2**

(12) # EUROPÄISCHE PATENTANMELDUNG

(21) Anmeldenummer: **86103880.0**

(22) Anmeldetag: **21.03.86**

(51) Int. Cl.⁴: **C 07 C 93/14**

(30) Priorität: **29.03.85 DE 3511544**

(43) Veröffentlichungstag der Anmeldung: **01.10.86 Patentblatt 86/40**

(84) Benannte Vertragsstaaten: **CH DE FR GB IT LI**

(71) Anmelder: **HOECHST AKTIENGESELLSCHAFT, Postfach 80 03 20, D-6230 Frankfurt am Main 80 (DE)**

(72) Erfinder: **Hunger, Klaus, Dr., Johann-Strauss-Strasse 35, D-6233 Kelkheim (Taunus) (DE)**
Erfinder: **Frölich, Heinrich, Dr., Obernhäuser Weg 6, D-6272 Niedernhausen/Taunus (DE)**
Erfinder: **Hertel, Hasso, Dr., Brunnenweg 10, D-6052 Mühlheim am Main (DE)**
Erfinder: **Habig, Kurt Conrad, Hundertmorgenring 98, D-6082 Mörfelden-Walldorf (DE)**

(54) **Verfahren zur Herstellung von 4,4'-Diaminodiphenylverbindungen und ihre Verwendung.**

(57) Die Erfindung betrifft ein Verfahren zur Herstellung von Verbindungen der Formel

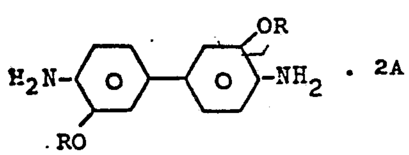

(I)

in der R eine n-Butyl-, Isopentyl- oder Phenylgruppe und A gleich Null oder ein Äquivalent einer anorganischen Säure ist, wobei ein n-Butyl-, Isopentyl- oder Phenylether des o-Nitrophenols in alkalischem Medium zum 2,2'-Alkoxy- bzw. 2,2'-Phenoxy-hydrazobenzol reduziert und anschließend im sauren Medium umgelagert wird. Die Verbindungen sind als Komponenten für die Herstellung von Farbstoffen und Pigmenten geeignet.

EP 0 196 025 A2

0196025



Verfahren zur Herstellung von 4,4'-Diaminodiphenylverbindungen und ihre Verwendung

Die Erfindung betrifft das Gebiet der Farbstoff- und Pigmentvorprodukte. Gegenstand der Erfindung ist ein Verfahren zur Herstellung einer 4,4'-Diaminodiphenylverbindung der allgemeinen Formel I,

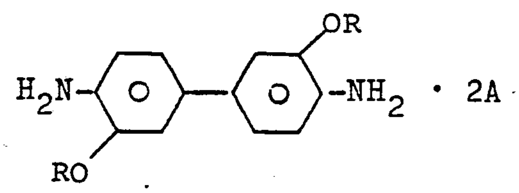

(I)

worin R eine n-Butyl-, Isopentyl- oder Phenylgruppe und A = 0 oder ein Äquivalent einer anorganischen Säure ist, dadurch gekennzeichnet, daß man eine Verbindung der Formel II,

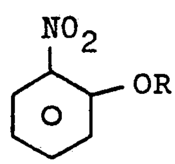

(II)

worin R die in der Verbindung der Formel I genannte Bedeutung hat, im alkalischen Medium zu der Verbindung der Formel III,

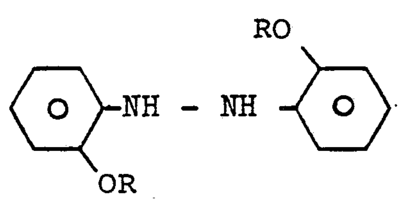

(III)

worin R die in der Verbindung der Formel I genannte Bedeutung hat, reduziert, anschließend mit einer Säure behandelt und das Umlagerungsprodukt in Form des freien Diamins, bzw. seines Salzes, der Formel I isoliert.

Ein Verfahren zur Herstellung der Verbindungen der Formel I ist bereits beschrieben (siehe N.A. Rosanelskaja, B.I. Stepanow, J. obsch. Chimii 31 758-764 (1961) bzw. die englische Übersetzung in J. General Chemistry of the UdSSR 31, 695-700 (1961)); es ist jedoch sehr aufwendig; Zunächst wird dabei die Disazoverbindung aus tetrazotiertem 3,3'-Dichlorbenzidin und p-Kresol hergestellt, die in einem zweiten Schritt mit dem Natriumalkoholat oder -phenolat NaOR, wobei R die in Formel I genannte Bedeutung hat, in Gegenwart von Kupferacetat in dem entsprechenden Alkohol (ROH) und einem organischen Lösemittel umgesetzt wird.

In einem weiteren Schritt wird die Disazoverbindung durch Zinn-II-chlorid reduktiv zu der Verbindung der Formel I gespalten.

Im Gegensatz zu den bekannten technisch äußerst aufwendigen Verfahren geht das erfindungsgemäße Verfahren von den leicht zugänglichen o-Nitroalkoxybenzolen bzw. dem o-Nitro-phenoxybenzol aus.

Die Reduktion der Nitrobenzolderivate der Formel II kann gemäß der vorliegenden Erfindung analog bekannter Reduktionsverfahren erfolgen, beispielsweise im wäßrigalkalischen Milieu mit Zinkstaub, Natriumamalgam, Wasserstoff in Gegenwart von Metallkatalysatoren, insbesondere Pd/C-Katalysatoren, oder elektrolytisch. Bevorzugt ist die Reduktion mit Zinkstaub in Natronlauge.

Die Reduktion kann in homogener Lösung oder im Zweiphasensystem unter Zusatz von Lösungsvermittlern, wie Alkoholen oder Kohlenwasserstoffen, durchgeführt werden. Neben dem gewünschten Hydrazobenzolderivat der Formel III tritt bei der Reduktion gewöhnlich als Nebenprodukt die der Nitroverbindung der Formel II entsprechende Aminoverbindung auf. Das Nebenprodukt wird vorteilhaft vor der Weiterverarbeitung des Hydrazobenzolderivates abgetrennt, was meist in einfacher Weise durch Extraktion einer Lösung des Reaktionsgemisches in einem organischen Lösungsmittel mit verdünnter Salzsäure möglich ist. Das erhaltene Hydrazobenzolderivat wird anschließend im sauren Medium analog bekannter Methoden umgelagert. Als Mittel zur Umlagerung sind gewöhnlich starke anorganische Säuren in wäßriger oder wäßrigalkoholischer Lösung geeignet. Auch Lösungen von Chlorwasserstoff in organischen Lösungsmitteln oder starke organische Säuren kommen in Frage. Von besonderem Interesse ist die Methode, bei der die Lösung der Hydrazoverbindung mit einem organischen Lösungsmittel wie Ether, Toluol, Chlorbenzol, Solventnaphtha oder dergleichen mit wäßrigen Mineralsäuren wie Halogenwasserstoffsäuren, Schwefelsäure, Phosphorsäure oder Salpetersäure verrührt wird.

Vorzugsweise wird das Hydrazobenzolderivat oder eine Lösung desselben in einem organischen Lösungsmittel mit Salzsäure oder wäßriger Schwefelsäure bei 5 bis 50°C, insbesondere bei 15 bis 30°C, behandelt. Die Reaktionszeit hängt vom pH-Wert und dem jeweiligen Hydrazobenzolderivat ab.

Das gewünschte Umlagerungsprodukt kann aus dem Rohproduktgemisch, gegebenenfalls nach Reinigungsschritten wie Filtration, Auswaschen mit organischen Lösungsmitteln, Umlösen oder Umkristallisieren in Form der 4,4'-Diaminodiphenylverbindung der Formel I, in der A ein Äquivalent der anorganischen Säure bei der Umlagerungsreaktion ist, isoliert werden.

Alternativ kann das Rohproduktgemisch der Umlagerung, gegebenenfalls nach obengenannten Reinigungsschritten, neutralisiert und das gewünschte Produkt als freies Diamin der Formel I (A=O) erhalten werden. In Einzelfällen ist es sinnvoll, das Umlagerungsprodukt in der Form des freien Diamins zu reinigen und anschließend mit der gleichen Säure wie bei der Umlagerung oder einer anderen geeigneten Säure zu Salzen der Formel I (A= anorganische Säure) umzusetzen und zu isolieren.

Neben dem oben beschriebenen neuen Herstellungsverfahren für die Verbindungen der Formel I wurde überraschenderweise gefunden, daß die Verbindungen der Formel I vorteilhaft zur Herstellung von Farbmitteln eingesetzt werden können.

Gegenstand der Erfindung ist deshalb auch die Verwendung der Verbindungen der Formel I zur Herstellung von Farbmitteln.

Bevorzugt werden die genannten Verbindungen zur Herstellung von Disazoverbindungen für Farbmittel verwendet. Als Farbmittel seien beispielsweise genannt: Farbstoffe wie Direktfarbstoffe, anionische Farbstoffe (Säurefarbstoffe), Lederfarbstoffe, kationische Farbstoffe, Entwicklungsfarbstoffe, Schwefelfarbstoffe, ferner Disazopigmente.

Für die Herstellung von Disazoverbindungen werden die erfindungsgemäßen Diamine analog bekannter Methoden, beispielsweise mit Natriumnitrit in wäßrig-salzsaurer Lösung, mit Nitrosylschwefelsäure in schwefelsaurer Lösung oder in organischen Lösemitteln mit aliphatischen organischen Nitriten bisdiazotiert und anschließend mit 2 Äquivalenten einer Kupplungskomponente umgesetzt.

Als Kupplungskomponenten sind beispielsweise Hydroxynaphthaline und alle sich davon ableitenden Verbindungen, wie Hydroxynaphthalinsulfonsäuren, Hydroxyaminonaphthalinsulfonsäuren, 2-Hydroxynaphthalin-3-carbonsäure, 2-Hydroxynaphthalin-3-carbonsäurearylide, aber auch methylen-aktive Verbindungen, wie Acetessigsäurearylide oder Malonsäuredianilide und heterocyclische "ankuppelbare" Verbindungen, wie Barbitursäuren, 2,4-Dihydroxychinolin oder Pyridone, wie 4-Methyl-5-cyanpyridon-2, geeignet.

Aber auch als Bestandteil von bifunktionellen Kupplungskomponenten, die man beispielsweise durch Bisdiketenisie-

rung der Diaminodiphenylderivate erhält und für Disazopigmente und -farbstoffe verwendet, sind die Verbindungen der Formel I geeignet.

Bei der Verwendung der Verbindungen der Formel I als Färbebasen für die Naphthol-AS-Färberei werden verbesserte Naßechtheiten der erhaltenen Färbungen erzielt.

Nach Diazotierung und Kupplung zu Disazodirektfarbstoffen lassen sich Farbstoffe erhalten, die Baumwolle mit z.T. überraschend hoher Farbstärke färben.

Die folgenden Beispiele sollen das Verfahren näher beschreiben. Der Mutagenitätstest wurde nach Ames mit Salmonella typhimurium (Stämme TA 98, TA 100, TA 1538, TA 1537, TA 1535) ohne und in Gegenwart von S-9-Mix durchgeführt (B.N. Ames et al., Mut. Res. 31, 347-364 (1975)).

Beispiel 1

In einer Eisenapparatur mit Ankerrührer, Heiz- und Kühlvorrichtung werden
293 g 2-n-Butoxy-1-nitrobenzol,
300 g Solventnaphtha und
220 g Zinkstaub
vorgelegt.
Unter Rühren wird das Gemisch auf 80°C erhitzt und zunächst 23 g 50 %ige Natronlauge und dann 21 g Wasser unter zeitweiser Kühlung zugegeben. Zur Vervollständigung der Reaktion wird bei 70 bis 80°C, eventuell unter Zugabe von weiterem Zinkstaub, nachgerührt, bis eine Tüpfelprobe der Reaktionslösung einen farblosen Auslauf zeigt. Durch einen weiteren Zusatz von Wasser erfolgt dann eine Körnung des Zinkoxidhydrats, welches durch Filtration von der organischen Phase abgetrennt wird. Die organische Phase enthält neben dem 2,2'-Di-n-butoxy-hydrazobenzol das als Nebenprodukt gebildete 2-n-Butoxy-1-aminobenzol. Das Nebenprodukt

wird mit verdünnter Salzsäure extrahiert. Die organische Phase gibt man darauf bei 8 - 10°C zu 20 %iger Salzsäure und rührt bei 15 - 20°C nach, bis die Umlagerung abgeschlossen ist (Probe: Keine Hydrazoverbindung mehr nachweisbar). Das Rohprodukt wird abfiltriert, in heißem Wasser gelöst und unter Zusatz von konzentrierter Salzsäure wieder ausgefällt. Man erhält 161 g 3,3'-Di-n-butoxy-4,4'-diaminodiphenyl-dichlorhydrat mit einem Diazotierungsgrad von 98 %, Schmelzpunkt 243°C.

Die freie Base wird nach Neutralisieren des oben erhaltenen Produkts mit wäßriger Ammoniaklösung, Extraktion mit heißem Xylol und anschließendem Kaltrühren erhalten. Schmelzpunkt der freien Base: 73°C. Die Verbindung ist im Mutagenitätstest nach Ames negativ.

Beispiel 2

In einer Eisenapparatur mit Ankerrührer, Heiz- und Kühlvorrichtung werden
508 g 2-Phenoxy-1-nitrobenzol,
460 g Solventnaphtha und
350 g Zinkstaub
vorgelegt.
Unter Rühren wird das Gemisch auf 80°C erhitzt und die Reduktion durch Zugabe von 32 g 50 %iger Natronlauge und dann von 28 g Wasser unter zeitweiser Kühlung kontrolliert durchgeführt. Zur Vervollständigung der Reduktion wird bei 70 bis 80°C unter portionsweiser Zugabe von weiterem Zinkstaub (ca. 70 g) nachgerührt, bis die Tüpfelprobe der Reaktionslösung einen farblosen Auslauf zeigt. Durch weiteren Zusatz von Wasser erfolgt dann eine Körnung des Zinkoxidhydrats, welches durch Filtration von der organischen Phase abgetrennt wird. Die organische Phase, die als Reduktionsprodukt 2,2'-Diphenoxy -hydrazobenzol enthält, läßt man darauf bei 35-40°C in 30 %ige Schwefelsäure einlaufen und rührt so lange nach bis die Umlagerung abgeschlossen ist (Probe:

Keine Hydrazoverbindung mehr nachweisbar). Das Rohprodukt wird abfiltriert und mit wäßriger Ammoniaklösung neutralisiert. Die erhaltene wäßrige Suspension wird filtriert und der Feststoff aus Ethanol umkristallisiert. Man erhält 201 g 3,3'-Diphenoxy-4,4'-diaminodiphenyl. Zweimaliges Umkristallisieren aus Xylol ergab den Schmelzpunkt 128°C. Die Verbindung ist im Mutagenitätstest nach Ames negativ.

PATENTANSPRÜCHE:

1. Verfahren zur Herstellung einer Verbindung der allgemeinen Formel I,

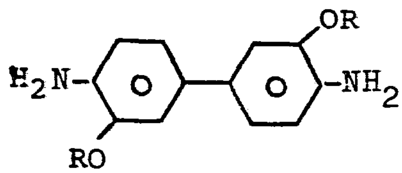 · 2A (I)

worin R eine n-Butyl-, Isopentyl- oder Phenylgruppe und A gleich Null oder ein Äquivalent einer anorganischen Säure ist, dadurch gekennzeichnet, daß man eine Verbindung der Formel II,

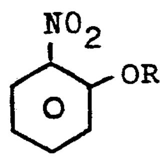 (II)

worin R die in der Verbindung der Formel I genannte Bedeutung hat, im alkalischen Medium zu der Verbindung der Formel III,

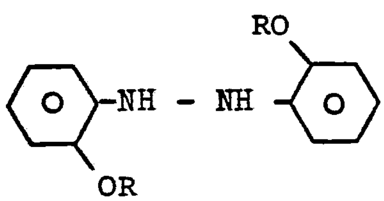 (III)

worin R die in der Verbindung der Formel I genannte Bedeutung hat, reduziert, anschließend mit einer Säure behandelt und das Umlagerungsprodukt in Form des freien Diamins, bzw. seines Salzes, der Formel I isoliert.

2. Verfahren nach Anspruch 1, dadurch gekennzeichnet, daß die anorganische Säure in Formel I Salz- oder Schwefelsäure ist.

3. Verfahren nach Anspruch 1 oder 2, dadurch gekennzeichnet, daß die Säure zur Behandlung der Verbindung der Formel III Salz- oder Schwefelsäure ist.

4. Verfahren nach Anspruch 1, 2 oder 3, dadurch gekennzeichnet, daß die Verbindung der Formel II mit Zinkstaub in Alkalilauge reduziert wird.

5. Verfahren nach Anspruch 1, 2 oder 3, dadurch gekennzeichnet, daß die Reduktion der Verbindungen der Formel II mit Wasserstoff in Gegenwart von Pd/C-Katalysatoren erfolgt.

6. Verwendung der Verbindungen der Formel I nach Anspruch 1 zur Herstellung von Farbmitteln.

7. Verwendung gemäß Anspruch 6 zur Herstellung von Disazofarbstoffen.

8. Verwendung gemäß Anspruch 6 zur Herstellung von Disazopigmenten.

9. Verwendung gemäß Anspruch 6 als Tetrazokomponente zur Herstellung von Disazo-Direktfarbstoffen.

10. Verwendung gemäß Anspruch 6 als Färbebase zur Herstellung von Entwicklungsfarbstoffen nach der Eisfarbentechnik.